(19) Europäisches Patentamt
European Patent Office
Office européen des brevets

(11) Numéro de publication: **0 145 591 B1**

(12) **FASCICULE DE BREVET EUROPEEN**

(45) Date de publication du fascicule du brevet: **22.06.88**

(21) Numéro de dépôt: **84402477.8**

(22) Date de dépôt: **03.12.84**

(51) Int. Cl.⁴: **C 07 D 487/06,** A 61 K 31/415 // C07D209/08 ,(C07D487/06, 209:00, 209:00)

(54) **Dérivés de pyrrolo[3,2,1-hi]indole, leur préparation et leur application en thérapeutique.**

(30) Priorité: **12.12.83 FR 8319850**
**27.09.84 FR 8414839**

(43) Date de publication de la demande:
**19.06.85 Bulletin 85/25**

(45) Mention de la délivrance du brevet:
**22.06.88 Bulletin 88/25**

(84) Etats contractants désignés:
**AT BE CH DE FR GB IT LI LU NL SE**

(56) Documents cités:
**EP - A - 0 071 368**

**CHEMICAL ABSTRACTS, volume 96, no. 15, 12 avril 1982, pages 60-61, abrégé 115832x, Columbus, Ohio, US; H. DABIRE et al.: "Pre- and postsynaptic alpha-adrenoceptor blockade by (imidazolinyl-2)-2-benzodioxane 1-4(170 150): antagonistic action on the central effects of clonidine"**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Bigg, Dennis M., 5, Parc de Diane, F-78350 Jouy en Josas (FR)**
Inventeur: **Morel, Claude M., 25, rue Gabriel Péri Cressely, F-78470 Magny-les-Hameaux (FR)**
Inventeur: **Sevrin, Mireille, 73, rue Raymond Losserand, F-75014 Paris (FR)**

(74) Mandataire: **Ludwig, Jacques et al, SYNTHELABO Service Brevets 58, rue de la Glacière, F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 145 591 B1

ACTORUM AG

## Description

La présente invention a pour objet des dérivés de pyrrolo-indole, leur préparation et leur application en thérapeutique. Les composés de l'invention répondent à la formule I donnée en annexe, dans laquelle

R représente un atome d'hydrogène ou un radical $(C_1-C_4)$alkyle droit ou ramifié, et

$R_1$ et $R_2$ représentent chacun, indépendamment l'un de l'autre, un atome d'hydrogène, un atome d'halogène ou un radical $(C_1-C_4)$alkyle.

Les composés de l'invention existent sous la forme de racémates ou d'isomères optiquement actifs, et sous forme de bases ou de sels d'addition à des acides pharmaceutiquement acceptables.

On connaît déjà des composés ayant le même domaine d'activité que les composés de l'invention (antagonisme des récepteurs $\alpha_2$-adrénergiques), par exemple l'(imidazolyl-2)-2 benzodioxane-1,4, étudié par H. Dabiré et coll., Arch. Int. Pharmacodyn., 254, 252–270, (1981), ou des dérivés d'(imidazolyl-2)-2 benzofuranne décrits dans la demande de brevet européen 0071368.

Conformément à l'invention les composés (I) peuvent être préparés selon le schéma réactionnel donné en annexe. L'ester de départ (II) dans lequel R' est un alkyle, en particulier éthyle, peut être obtenu à partir de N-amino-indoline selon la méthode décrite par H. Rapoport et coll., J.A.C.S., (1958), 80, 5574–5, la N-amino-indoline elle-même étant préparée selon la méthode décrite par A.N. Kost et coll., C.A., 54, 19641.

Selon l'invention on hydrogène l'ester de départ (II) à l'aide d'acide chlorhydrique en présence d'étain à la température ambiante.

Puis

– soit on fait agire le composé (III) obtenu avec de l'éthylènediamine en présence de triméthylaluminium pour obtenir les composés (I) dans lesquels R est H,

– soit on alkyle le composé (III) par réaction avec un halogénure d'alkyle RX, en présence de diisopropylamine et de butyllithium, dans un solvant, et on fait réagir le composé alkylé obtenu (IV) avec de l'éthylènediamine en présence de triméthylaluminium, pour obtenir les composés (I) dans lesquels R est un alkyle.

Les exemples suivants illustrent l'invention:

Les spectres et les analyses IR et RMN confirment la structure des composés.

Exemple 1

(dihydro-4,5 1H-imidazolyl-2)-2 tétrahydro-1,2,4,5 pyrrolo[3,2,1-hi] indole et son fumarate.

1. Dans un ballon tricol de 1000 ml muni d'une agitation magnétique, d'une arrivée de chlorure d'hydrogène gazeux, d'un réfrigérant à air avec garde à chlorure de calcium, d'un thermomètre, et placé dans un bain de carboglace et d'alcool isopropylique, on introduit 15,8 g (0,073 mole) de dihydro-4,5 pyrrolo[3,2,1-hi] indolecarboxylate-2 d'éthyle avec 150 ml d'éthanol.

On refroidit le mélange à −20 °C et on condense le gaz chlorhydrique à cette température jusqu'à obtenir une solution. On ajoute alors en une seule fois 26,1 g (0,22 atome-gramme) d'étain en grenaille, on retire le bain froid et on maintient l'agitation pendant 20 heures à la température ambiante.

On obtient une suspension de couleur jaune, on la concentre au bain-marie et on la reprend avec 550 ml d'éthanol absolu. On refroidit le mélange, on y fait barboter de l'ammoniac jusqu'à pH = 9 à 10 pour précipiter les sels d'étain, on essore ces derniers, en les lavant à l'éthanol glacé, et on évapore à sec le filtrat obtenu. On soumet le résidu à une chromatographie sur colonne de silice en éluant avec du chlorure de méthylène. On recueille finalement 11,65 g d'un produit jaune huileux.

2. Dans un ballon de Keller de 100 ml muni d'une agitation magnétique, d'un réfrigérant à reflux avec garde à chlorure de calcium, d'un thermomètre, d'une arrivée d'argon, d'une ampoule à introduction et d'un appareil de Dean-Stark, on introduit sous argon, successivement, 16 ml de toluène, 10,3 ml (0,025 mole) de triméthylaluminium à 25,2% dans de l'hexane et, tout en refroidissant, 1,6 ml (soit 1,43 g ou 0,024 mole) d'éthylènediamine en solution dans 4,5 ml de toluène.

On agite le mélange pendant 5 minutes, puis on le chauffe à 50 °C et, à cette température, on ajoute 3,3 g (0,015 mole) du produit précédemment obtenu en solution dans 15 ml de toluène. Ensuite on chaffe au reflux pendant 6 heures, puis on laisse refroidir. Après avoir refroidi le mélange entre −10 et −15 °C, on l'hydrolyse avec 10,2 ml d'eau, tout en agitant, puis on l'extrait avec de l'acétate d'éthyle. On réunit les fractions organiques, on les lave avec une solution de chlorure de sodium, on les sèche, on les filtre et on les évapore. Il reste environ 3 g d'un solide gras de couleur jaune dont on prépare directement le fumarate. Pour cela on le reprend avec 50 ml d'éthanol, on filtre la solution et on lui ajoute une solution filtrée de 1,5 g (0,013 mole) d'acide fumarique dans 100 ml d'éthanol. On agite la solution obtenue, on la concentre à sec, on reprend le résidu avec de l'acétone, on le filtre, on le sèche sous vide et on le recristallise dans l'éthanol. On recueille 1,65 g du fumarate qui fond à 184,5–186 °C.

Exemple 2

méthyl-2 (dihydro-4,5 1H-imidazolyl-2)-2 tétrahydro-1,2,4,5 pyrrolo[3,2,1-hi] indole et son fumarate.

2.1. méthyl-2 tétrahydro-1,2,4,5 pyrrolo-[3,2,1-hi] indolecarboxylate-2 d'éthyle.

Dans un ballon de Keller de 250 ml, muni d'une agitation magnétique, d'un thermomètre, d'une arrivée d'argon, d'une ampoule à introduction et placé dans un bain froid, on introduit, sous argon 5,6 ml (0,04 mole) de diisopropylamine et 35 ml de tétrahydrofuranne (THF). On refroidit le mélange réactionnel à −70–75 °C puis on introduit,

en 20 mn, 25 ml (0,04 mole) de butyllithium en solution 1,6 molaire dans de l'hexane.

On maintient la température à −70–75 °C pendant 1 h et ajoute en 15 mn une solution de 7 g (0,0322 mole) de tétrahydro-1,2,4,5 pyrrolo[3,2,1-hi] indolecarboxylate-2 d'éthyle dans 25 ml de THF.

On maintient encore la température à −70–75 °C pendant 1 h et on ajoute alors en 20 mn une solution de 12,4 ml (0,2 mole) d'iodure de méthyle dans 20 ml de THF.

On maintient le mélange réactionnel à −70–75 °C pendant 1 h puis le laisse à la température ambiante pendant 3 h 30. On le verse dans de l'eau glacée. On l'extrait à l'éther diéthylique en présence d'une solution saturée de chlorure de sodium. On lave à l'eau et sèche sur $Na_2SO_4$. On sépare la phase organique. On évapore à siccité au bain-marie sous vide. On obtient une huile que l'on purifie par passage sur une colonne de silice avec comme éluant du chlorure de méthylène.

2.2. méthyl-2 (dihydro-4,5 1H-imidazolyl-2)-2 tétrahydro-1,2,4,5 pyrrolo[3,2,1-hi] indole et son fumarate.

Dans un ballon de Keller de 50 ml muni d'une agitation magnétique, d'un réfrigérant à reflux, d'un thermomètre, d'une arrivée d'argon, d'une ampoule à introduction et d'un appareil de Dean-Stark, on introduit sous argon, successivement, 10 ml de toluène, 5,4 ml (0,013 mole) de triméthylaluminium à 25,2% dans de l'hexane et, tout en refroidissant à 0 °C, 0,9 ml (soit 0,013 mole) d'éthylènediamine en solution dans 3 ml de toluène.

On agite le mélange pendant 10 minutes, puis on le chauffe à 50 °C et, à cette température, on ajoute 1,9 g (0,0082 mole) du produit précédemment obtenu en solution dans 10 ml de toluène. Ensuite on chauffe au reflux pendant 6 heures, puis on laisse refroidir. Après avoir refroidi le mélange entre −10 et −15 °C, on l'hydrolyse avec 5,4 ml d'eau, tout en agitant, puis on l'extrait avec de l'acétate d'éthyle. On réunit les fractions organiques, on les lave avec une solution de chlorure de sodium, on les sèche, on les filtre et on les évapore. Il reste un solide de couleur jaune dont on prépare directement le fumarate.

Pour cela on le reprend avec 25 ml d'éthanol, on filtre la solution et on lui ajoute une solution filtrée de 0,7 g (0,006 mole) d'acide fumarique dans 50 ml d'éthanol. On agite la solution obtenue, on la concentre à sec, on reprend le résidu avec de l'acétone, on le filtre, on le sèche sous vide et on le recristallise dans de l'éthanol. On recueille le fumarate qui fond à 192–194 °C.

Exemple 3

n-propyl-2 (dihydro-4,5 1H-imidazolyl-2)-2 méthyl-7 tétrahydro-1,2,4,5 pyrrolo[3,2,1-hi] indole et son fumarate.

3.1. méthyl-5 nitroso-1 dihydro-2,3 1H-indole

Dans un tricol de 500 ml muni d'un système d'agitation magnétique on introduit la méthyl-5 indoline [préparée selon G.W. Gribble et J.H. Hoffman, Synthesis, 1977, 859–860 à partir de l'indole décrit par J.E. Nordlander et al, J. Org. Chem. 46, 778–782 (1981)] à raison de 18 g (0,14 mole) et 300 ml d'acide sulfurique à 20%.

La solution est refroidie à 0 °C et on ajoute le nitrite de sodium (9,7 g – 0,14 mole) en solution dans de l'eau (30 ml). Le mélange réactionnel est maintenu à 0 °C lors de l'addition.

Après 30 minutes d'agitation à 0 °C, la phase aqueuse est extraite à l'éther (500 ml). La phase éthérée est lavée à l'eau, puis avec une solution saturée de NaCl et séchée sur sulfate de magnésium. Les solvants sont évaporés sous vide. Le composé obtenu est utilisé tel quel dans l'étape suivante.

3.2. méthyl-5 dihydro-2,3 1H-indoleamine-1

Dans un ballon tricol de 2 l muni d'un système d'agitation mécanique, d'un réfrigérant, d'une ampoule à addition, d'un thermomètre et sous argon, on introduit l'hydrure de lithium et d'aluminium (6,08 g – 0,16 mole) et le tétrahydrofuranne (THF) (300 ml). On ajoute à la suspension le composé obtenu précédemment (22 g – 0,14 mole) en solution dans du THF (300 ml). La température est maintenue à 35 °C.

Le mélange réactionnel est agité pendant 4 h à 20 °C puis hydrolysé ($H_2O$ · 10 ml). On ajoute alors 10 ml d'une solution NaOH 1N, puis 20 ml d'eau.

La suspension est agitée à 20 °C pendant 30 mn puis filtrée et rincée à l'éther.

La phase organique est séchée sur sulfate de sodium.

Les solvants sont évaporés sous vide.

Le composé obtenu est utilisé tel quel dans l'étape suivante.

3.3. [(méthyl-5 dihydro-2,3 1H-indolyl-1)imino]-2 propanoate d'éthyle

Dans un ballon monocol de 500 ml, muni d'un système d'agitation magnétique, d'un réfrigérant et sous argon, on introduit le composé précédent (20 g – 0,13 mole), le pyruvate d'éthyle (16,24 g – 0,14 mole), l'éthanol (200 ml) et l'acide acétique (0,5 ml).

Le mélange réactionnel est agité à 80 °C pendant 5 h.

L'éthanol est évaporé sous vide et le résidu est purifié par chromatographie sur colonne ($SiO_2$, éluant: cyclohexane 2/éther 1).

Le composé obtenu est utilisé tel quel dans l'étape suivante.

3.4. méthyl-7 dihydro-4,5 pyrrolo[3,2,1-hi] indolecarboxylate-2 d'éthyle.

Dans un ballon tricol de 25 ml muni d'un système d'agitation magnétique, d'un réfrigérant et sous argon, on introduit le composé obtenu précédemment (7 g – 0,028 mole), l'acide acétique (8 ml) et on ajoute l'éthérate de $BF_3$ (3,42 ml – 0,028 mole). Le mélange réactionnel est agité à 90 °C pendant 50 mn. On refroidit, on hydrolyse ($H_2O$ 40 ml). La phase aqueuse est extraite à l'éther (3 × 200 ml). La phase organique est lavée au bicarbonate de sodium, avec une solution de NaCl saturée, puis séchée sur sulfate de sodium.

Les solvants sont évaporés sous vide et le résidu est purifié par chromatographie sur colonne

[$SiO_2$; éluant: cyclohexane/ether (2/1)].

L'ester (II) obtenu est utilisé dans l'étape suivante.

3.5. méthyl-7 tétrahydro-1,2,4,5 pyrrolo-[3,2,1-hi] indolecarboxylate-2 d'éthyle.

Dans un tricol de 50 ml muni d'un système d'agitation magnétique, d'un réfrigérant, on introduit l'éthanol (21 ml). L'éthanol est saturé en acide chlorhydrique à −10 °C. On ajoute l'ester obtenu précédemment (2,4 g – 0,011 mole), puis on ajoute l'étain (3,8 g – 0,032 at g). Le mélange réactionnel est agité à +20 °C pendant 8 h. Le solvant est évaporé sous vide et le résidu est dissous dans de l'éthanol (50 ml). On sature en ammoniac jusqu'à pH = 9. La suspension est filtrée et l'éthanol est évaporé sous vide. Le résidu est dissous dans de l'eau, la phase aqueuse est extraite à l'éther (300 ml); la phase éthérée est lavée avec une solution de chlorure de sodium saturée puis séchée sur sulfate de sodium.

On obtient l'ester (III).

3.6. n-propyl-2 méthyl-7 tétrahydro-1,2,4,5 pyrrolo[3,2,1-hi] indolecarboxylate-2 d'éthyle.

Dans un ballon tricol de 500 ml, muni d'un système d'agitation magnétique et sous argon, on introduit la diisopropylamine (0,600 g – 0,006 mole) et le THF (5 ml). La solution est refroidie à −78 °C et on ajoute le n-butyllithium dans de l'hexane (3,75 ml – 0,006 mole). La solution est agitée à −78 °C, pendant 30 mn, puis on ajoute l'ester obtenu précédemment (III) (1,15 g – 0,005 mole) dans le THF (10 ml). Le mélange réactionnel est agité à −78 °C pendant 1 h (coloration brune). On ajoute l'iodo-1 propane (1,02 g–0,006 mole) dans le THF (5 ml).

Le mélange réactionnel est agité à −78 °C pendant 1 h puis à 20 °C pendant 1 h. On hydrolyse le mélange ($H_2O$ – 10 ml). La phase aqueuse est extraite à l'éther.

La phase éthérée est lavée à l'eau, avec une solution de chlorure de sodium saturée puis séchée sur sulfate de sodium. L'ester obtenu (IV) est utilisé dans l'étape suivante.

3.7. n-propyl-2 (dihydro-4,5 1H-imidazolyl-2)-2 méthyl-7 tétrahydro-1,2,4,5 pyrrolo-[3,2,1-hi] indole et son fumarate.

a. Dans un ballon tricol de 50 ml muni d'un système d'agitation magnétique, d'un réfrigérant et sous argon, on introduit le toluène (10 ml) et le triméthylaluminium à 25% dans de l'hexane (4,5 ml – 0,011 mole). La solution est refroidie à −10 °C puis on ajoute l'éthylènediamine (0,640 g – 0,011 mole) dans le toluène (5 ml). On laisse revenir le mélange réactionnel à 20 °C et on ajoute l'ester (IV) obtenu sous 3.6 (1,22 g – 0,0044 mole) dans le toluène (10 ml).

Le mélange réactionnel est agité à 110 °C pendant 4 h. On refroidit à −10 °C, puis on hydrolyse ($H_2O$ – 5 ml) et on ajoute l'acétate d'éthyle (50 ml).

On agite 30 mn à 0 °C puis on filtre. On sèche sur $Na_2SO_4$. Les solvants sont évaporés sous vide. On prépare directement le furmarate.

b. Dans un ballon monocol de 250 ml, muni d'un système d'agitation magnétique, on introduit le composé obtenu sous a dans l'éthanol (20 ml) et on ajoute l'acide fumarique (0,500 g – 0,0042 mole) dans l'éthanol (30 ml). La solution est agitée pendant 30 mn, puis l'éthanol est évaporé sous vide. On reprend le résidu avec de l'éther. On le filtre, on le sèche sous vide et on le recristallise dans de l'éthanol. On recueille le fumarate qui fond à 178–180 °C.

Tableau

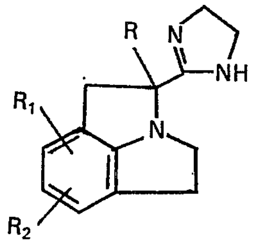

(1)

| Composé | $R_1$ | $R_2$ | R | sel | F(°C) |
|---|---|---|---|---|---|
| 1 | H | H | H | fumarate | 184–186 |
| | | | | benzoate | 146–148 |
| 2 | H | H | $CH_3$ | fumarate | 192–194 |
| 3 | H | H | $C_2H_5$ | fumarate | 162–164 |
| 4 | H | H | $nC_3H_7$ | fumarate | 202–204 |
| 5 | H | H | $nC_4H_9$ | fumarate | 171–173 |
| 6 | 8–Cl | H | H | fumarate | 195–196 |
| 7 | 8–Cl | H | $CH_3$ | fumarate | 94–99 |
| 8 | 8–Cl | H | $nC_3H_7$ | fumarate | 202–204 |
| 9 | 7–F | H | H | fumarate | 137–141 |
| 10 | 7–F | H | $CH_3$ | fumarate | 147–150 |
| 11 | 7–F | H | $nC_3H_7$ | fumarate | 200–203 |
| 12 | 7–$CH_3$ | H | H | fumarate | 168–172 |

Tableau (suite)

| Composé | $R_1$ | $R_2$ | R | sel | F(°C) |
|---|---|---|---|---|---|
| 13 | 7–$CH_3$ | H | $nC_3H_7$ | fumarate | 178–180 |
| 14 | 6–$CH_3$ | 8–$CH_3$ | H | fumarate | 217–219 |
| 15 | 6–$CH_3$ | 8–$CH_3$ | $CH_3$ | fumarate | 207–210 |
| 16 | 6–$CH_3$ | 8–$CH_3$ | $nC_3H_7$ | fumarate | 180–185 |

Les composés de l'invention ont été soumis à des essais pharmacologiques qui ont montré leur intérêt en tant que $\alpha_2$-antagonistes.

A cet effet les composés ont été étudiés dans le test de potentialité et de sélectivité des antagonistes à l'égard des récepteurs $\alpha_2$ in vitro.

La détermination de la valeur $pA_2$ à l'égard des effets inhibiteurs de la clonidine, $\alpha_2$-agoniste bien connu, a eu lieu sur le vas deferens du rat stimulé à une fréquence de 0,1 Hz en présence de 30 nM de prazosine et de 1 µM de cocaïne, selon la méthode décrite par G.M. Drew [European Journal of Pharmacology, 42 (1977), 123–130].

Les $pA_2$ des composés de l'invention vont de 6 à 9,5.

Les composés de l'invention sont des $\alpha_2$-antagonistes puissants qui peuvent être utilisés pour le traitement de la dépression (soit seuls, soit en association avec un produit qui inhibe les mécanismes de captation neuronale), le traitement de l'hypotension, le traitement de l'ileum paralytique post-opératoire, le traitement de l'asthme et de l'obésité.

Les compositions pharmaceutiques peuvent être sous une forme appropriée pour l'administration par voie orale, rectale ou parentérale; par exemple sous la forme de capsules, comprimés, granulés, gélules ou solutés liquides, sirops ou suspensions buvables, et contenir les excipients appropriés.

La posologie quotidienne peut aller de 0,1 à 10 mg/kg p.o.

Annexe

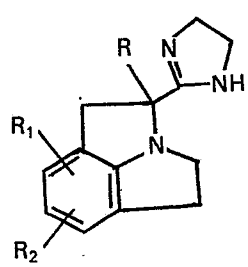

(I)

Schéma réactionnel

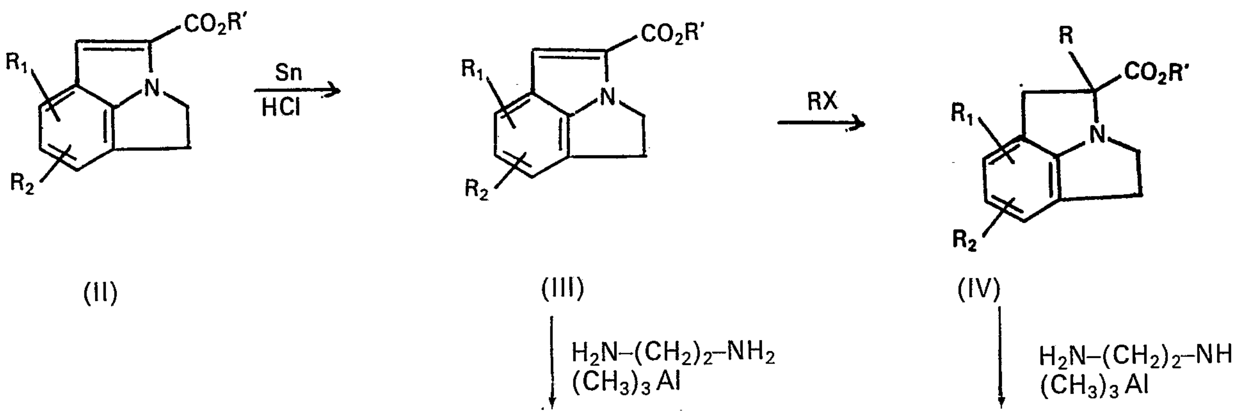


(II) (III) (IV)

(I) où R = H (I) où R = alkyle

R' = alkyle de préférence éthyle
X = halogène de préférence I

**Revendications pour les états contractants: BE, CH, DE, FR, GB, IT, LI, LU, NL, SE**

1. Dérivés de pyrrolo[3,2,1-hi] indole, sous la forme de racémates ou d'isomères optiquement actifs, répondant à la formule I

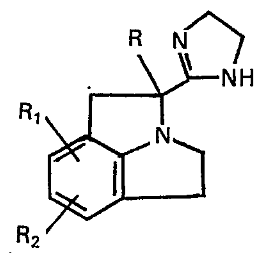

(I)

dans laquelle

R représente un atome d'hydrogène ou un radical ($C_{1-4}$) alkyle droit ou ramifié, et

$R_1$ et $R_2$ représentent chacun, indépendamment l'un de l'autre, un atome d'hydrogène, un atome d'halogène ou un radical ($C_{1-4}$)alkyle.

2. Composés selon la revendication 1, dans lesquels R est le radical méthyle, éthyle, n-propyle ou n-butyle.

3. Composés selon la revendication 1 ou la revendication 2, dans lesquels $R_1$ et $R_2$ sont chacun indépendamment l'un de l'autre un atome d'hydrogène, le radical méthyle, éthyle, n-propyle ou n-butyle, un atome de chlore ou de fluor.

4. Procédé de préparation des composés tels que spécifiés dans l'une quelconque des revendications 1 à 3, procédé caractérisé par le fait que l'on hydrogène un ester de formule II

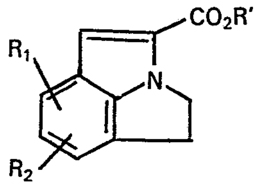

(II)

à l'aide d'acide chlorhydrique, en présence d'étain, à la température ambiante, puis

– soit on fait réagir le composé (III) obtenu de formule III

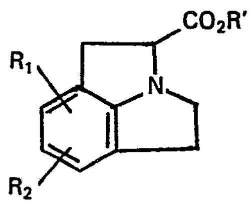

(III)

avec de l'éthylènediamine en présence de triméthylaluminium, pour obtenir les composés (I) dans lesquels R est H,

– soit on alkyle le composé (III) obtenu de formule III

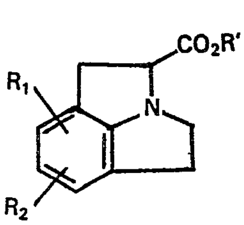

(III)

en le faisant réagir avec un halogènure d'alkyle RX et on fait ensuite réagir le composé (IV) obtenu de formule IV

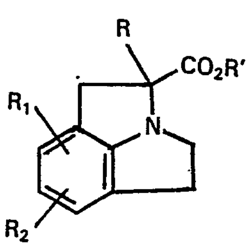

(IV)

avec de l'éthylènediamine, en présence de triméthylaluminium, pour obtenir les composés (I) dans lesquels R est un alkyle, les significations des radicaux R, $R_1$ et $R_2$ étant données dans la revendication 1, et X étant un halogène et R' un alkyle.

5. Médicament caractérisé en ce qu'il est constitué par un composé tel que spécifié dans l'une quelconque des revendications 1 à 3.

6. Composition pharmaceutique caractérisée en ce qu'elle contient un composé tel que spécifié dans l'une quelconque des revendications 1 à 3, en association avec tout excipient approprié.

**Revendication pour l'état contractant: AT**

Procédé de préparation de dérivés de pyrrolo[3,2,1-hi] indole, sous la forme de racémates ou d'isomères optiquement actifs, répondant à la formule I

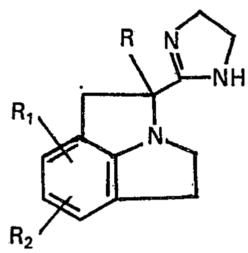

(I)

dans laquelle

R représente un atome d'hydrogène ou un radical ($C_{1-4}$)alkyle droit ou ramifié, et

$R_1$ et $R_2$ représentent chacun, indépendamment l'un de l'autre, un atome d'hydrogène, un atome d'halogène ou un radical ($C_{1-4}$)alkyle, procédé caractérisé par le fait que l'on hydrogène un ester de formule II

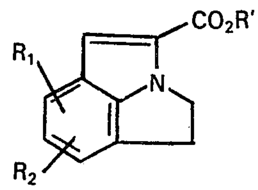

(II)

à l'aide d'acide chlorhydrique, en présence d'étain, à la température ambiante, puis

– soit on fait réagir le composé (III) obtenu de formule III

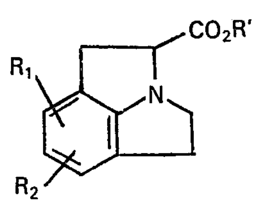

(III)

avec de l'éthylènediamine en présence de triméthylaluminium, pour obtenir les composés (I) dans lesquels R est H,

– soit on alkyle le composé (III) obtenu de formule III

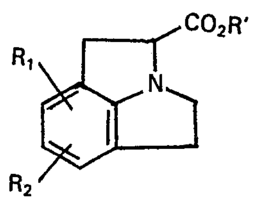

(III)

en le faisant réagir avec un halogénure d'alkyle RX et on fait ensuite réagir le composé (IV) obtenu de formule IV

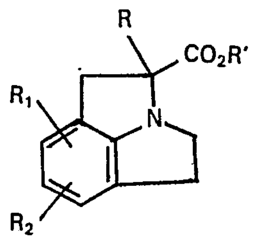

(IV)

avec de l'éthylènediamine, en présence de triméthylaluminium, pour obtenir les composés (I) dans lesquels R est un alkyle, les significations des radicaux R, $R_1$ et $R_2$ étant données ci-dessus, et X étant un halogène et R' un alkyle.

**Patentansprüche für die Vertragsstaaten BE, CH, DE, FR, GB, IT, LI, LU, NL, SE**

1. Pyrrolo [3,2,1-hi] indol-Derivate in Form der Racemate oder optisch aktiven Isomeren, entsprechend der Formel I

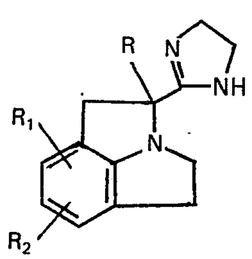

(I)

worin R für ein Wasserstoffatom oder einen geradkettigen oder verzweigten $(C_{1-4})$-Alkylrest und $R_1$ und $R_2$ jeweils unabhängig voneinander für ein Wasserstoffatom, ein Halogenatom oder einen $(C_{1-4})$-Alkylrest steht.

2. Verbindungen nach Anspruch 1, bei welchen R für den Methyl-, Ethyl, n-Propyl- oder n-Butylrest steht.

3. Verbindungen nach Anspruch 1 oder Anspruch 2, bei welchen $R_1$ und $R_2$ jeweils unabhängig voneinander für ein Wasserstoffatom, den Methyl-, Ethyl-, n-Propyl- oder n-Butylrest, ein Chlor- oder Fluoratom stehen.

4. Verfahren zur Herstellung der in einem der Ansprüche 1 bis 3 dargelegten Verbindungen, dadurch gekennzeichnet, dass man einen Ester der Formel II

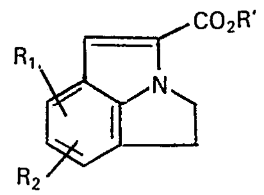

(II)

mit Hilfe von Chlorwasserstoffsäure in Gegenwart von Zinn bei Raumtemperatur hydriert und dann entweder

– die erhaltene Verbindung (III) der Formel III

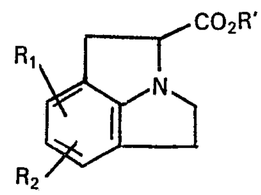

(III)

mit Ethylendiamin in Gegenwart von Trimethylaluminium zur Gewinnung der Verbindungen (I), in welchen R für H steht, reagieren lässt, oder

– die erhaltene Verbindung (III) der Formel III

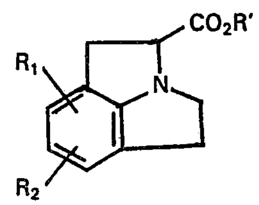

(III)

alkyliert, indem man sie mit einem Alkylhalogenid RX umsetzt und anschliessend die erhaltene Verbindung (IV) der Formel IV

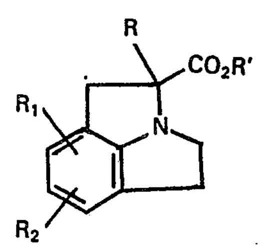

(IV)

mit Ethylendiamin in Gegenwart von Trimethylaluminium zur Gewinnung der Verbindung (I), in welcher R für ein Alkyl steht, reagieren lässt, wobei die Bedeutung der Reste R, $R_1$ und $R_2$ in Anspruch 1 angegeben sind und X für ein Halogen und R' für ein Alkyl steht.

5. Arzneimittel, dadurch gekennzeichnet, dass es aus einer in irgendeinem der Ansprüche 1 bis 3 angegebenen Verbindung besteht.

6. Pharmazeutische Zusammensetzung, dadurch gekennzeichnet, dass sie eine in irgendeinem der Ansprüche 1 bis 3 angegebene Verbindung in Kombination mit irgendeinem geeigneten Bindemittel enthält.

**Patentanspruch für den Vertragsstaat AT**

Verfahren zur Herstellung von Pyrrolo [3-2-1-hi] indol-Derivaten in der Form der Racemate

oder optisch aktiven Isomeren, entsprechend der Formel I

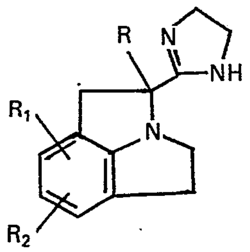

(I)

worin R für ein Wasserstoffatom oder einen geradkettigen oder verzweigten $(C_{1-4})$-Alkylrest und $R_1$ und $R_2$ jeweils unabhängig voneinander für ein Wasserstoffatom, ein Halogenatom oder einen $(C_{1-4}$-Alkylrest steht, dadurch gekennzeichnet, dass man einen Ester der Formel II

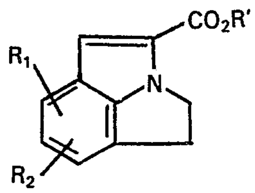

(II)

mit Hilfe von Chlorwasserstoffsäure in Gegenwart von Zinn bei Raumtemperatur hydriert und dann entweder

– die erhaltene Verbindung (III) der Formel III

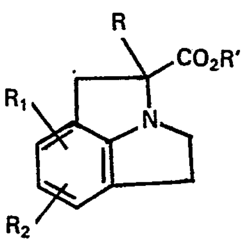

(III)

mit Ethylendiamin in Gegenwart von Trimethylaluminium zur Gewinnung der Verbindungen (I), in welcher R für H steht, reagieren lässt, oder

– die erhaltene Verbindung (III) der Formel III

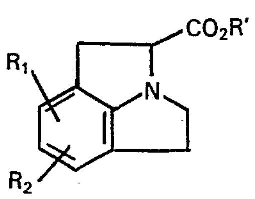

(III)

alkyliert, indem man sie mit einem Alkylhalogenid RX umsetzt und anschliessend die erhaltene Verbindung (IV) der Formel IV

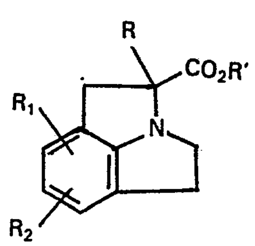

(IV)

mit Ethylendiamin in Gegenwart von Trimethylaluminium zur Gewinnung der Verbindung (I), in welchen R für ein Alkyl steht, reagieren lässt, wobei die Bedeutung der Reste R, $R_1$ und $R_2$ in Anspruch 1 angegeben sind und X für ein Halogen und R' für ein Alkyl steht.

**Claims for the contracting states: BE, CH, DE, FR, GB, IT, LI, LU, NL, SE**

1. Pyrrolo[3,2,1-hi]indole derivatives, in the form of racemates or optically active isomers, corresponding to the formula I

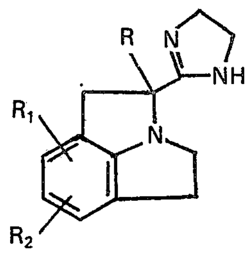

(I)

in which

R denotes a hydrogen atom or a linear or branched $C_{1-4}$-alkyl radical, and

$R_1$ and $R_2$ each denote, independently of each other, a hydrogen atom, halogen atom or $C_{1-4}$-alkyl radical,

2. Compounds according to Claim 1, in which R is a methyl, ethyl, n-propyl or n-butyl radical.

3. Compounds according to Claim 1 or Claim 2, in which $R_1$ and $R_2$ are, independently of each other, a hydrogen atom, a methyl, ethyl, n-propyl or n-butyl radical, or a chlorine or fluorine atom.

4. Process for the preparation of the compounds as specified in any one of Claims 1 to 3, which process is characterised in that an ester of formula II

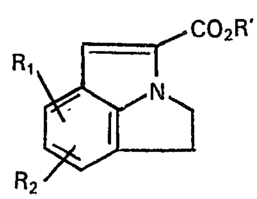

(II)

is hydrogenated by means of hydrochloric acid in the presence of tin at room temperature, and then

– either the compound (III) obtained of formula III

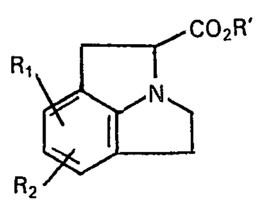

(III)

is reacted with ethylenediamine in the presence of trimethylaluminium to obtain the compounds (I) in which R is H

– or the compound (III) obtained of formula III

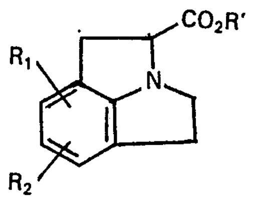

(III)

is alkylated by reacting it with an alkyl halide RX, and the compound (IV) obtained of formula IV

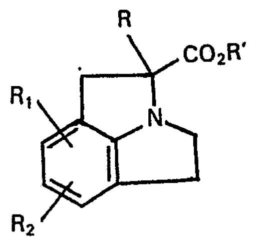

(IV)

is then reacted with ethylenediamine in the presence of trimethylaluminium to obtain the compounds (I) in which R is an alkyl group, the significance of the radicals R, $R_1$ and $R_2$ being given in Claim 1, and

X bein a halogen and R′ an alkyl group.

5. Drug, characterised in that it consists of a compound as specified in any one of Claims 1 to 3.

6. Pharmaceutical composition, characterised in that it contains a compound as specified in any one of Claims 1 to 3, in association with any suitable excipient.

**Claim for the contracting state: AT**

Process for the preparation of pyrrolo[3,2,1-hi]indole derivatives, in the form of racemates or optically active isomers, corresponding to the formula

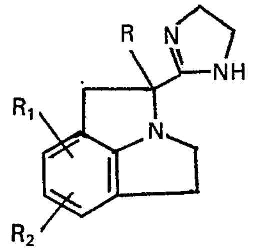

(I)

in which

R denotes a hydrogen atom or a linear or branched $C_{1-4}$-alkyl radical, and

$R_1$ and $R_2$ each denote, independently of each other, a hydrogen atom, halogen atom or $C_{1-4}$-alkyl radical, which process is characterised in that an ester of formula II

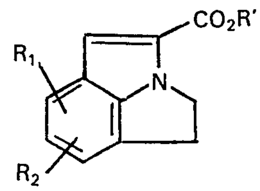

(II)

is hydrogenated by means of hydrochloric acid in the presence of tin at room temperature, and then

– either the compound (III) obtained of formula

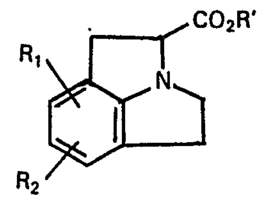

(III)

is reacted with ethylenediamine in the presence of trimethylaluminium to obtain the compounds (I) in which R is H,

– or the compound (III) obtained of formula III

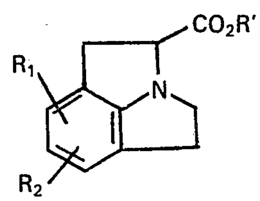

(III)

is alkylated by reacting it with an alkyl halide RX, and the compound (IV) obtained of formula IV

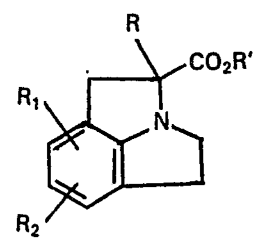

(IV)

is then reacted with ethylenediamine in the presence of trimethylaluminium to obtain the compounds (I) in which R is an alkyl group, the significance of the radicals R, $R_1$ and $R_2$ being given above, and X being a halogen and R′ an alkyl group.